# EUROPEAN PATENT APPLICATION

(11) **EP 4 412 252 A1**
(43) Date of publication of application: **07.08.2024**
(21) Application number: 22876071.6
(22) Date of filing: 22.09.2022
(51) Int. Cl.: H04R 17/00, A61B 8/00, C08G 59/20, C08K 5/18, C08L 63/00

(54) **ACOUSTIC MATCHING LAYERED MATERIAL, ACOUSTIC MATCHING SHEET, COMPOSITION FOR ACOUSTIC MATCHING LAYERED MATERIAL, ACOUSTIC WAVE PROBE, ACOUSTIC WAVE MEASUREMENT DEVICE, AND METHOD FOR MANUFACTURING ACOUSTIC WAVE PROBE**

(30) Priority: 30.09.2021 JP 2021161985
(71) Applicant: FUJIFILM Corporation, Tokyo 106-8620 (JP)
(72) Inventor: FURUKAWA, Kazushi, Ashigarakami-gun, Kanagawa 258-8577 (JP); NAKAI, Yoshihiro, Ashigarakami-gun, Kanagawa 258-8577 (JP)
(74) Representative: HGF
(86) International application number: PCT/JP2022/035513
(87) International publication number: WO 2023/054204

(57) **Abstract**

Provided are an acoustic matching layer material containing a curing reaction product obtained by reacting an epoxy resin (A) having an epoxy equivalent weight of 140 or less with at least one of a curing agent (B-1) which is a monomer or a curing catalyst (B-2), and metal particles (C) having a density of 4.5 g/cm³ or more and less than 10 g/cm³ at 20°C, in which a content of particles (D) having a density of less than 4.5 g/cm³ at 20°C is less than 5% by mass; an acoustic matching sheet; a composition for an acoustic matching layer material; an acoustic wave probe; an acoustic wave measurement apparatus; and a manufacturing method of an acoustic wave probe.

## Description

### BACKGROUND OF THE INVENTION

### 1. Field of the Invention

The present invention relates to an acoustic matching layer material, an acoustic matching sheet, a composition for an acoustic matching layer material, an acoustic wave probe, an acoustic wave measurement apparatus, and a manufacturing method of an acoustic wave probe.

### 2. Description of the Related Art

In an acoustic wave measurement apparatus, an acoustic wave probe is used which irradiates a test object such as a living body with an acoustic wave, receives a reflected wave (echo) therefrom, and outputs a signal. The reflected wave received by this acoustic wave probe is converted into an electric signal which is displayed as an image. Therefore, by using the acoustic wave probe, it is possible to visualize and observe an inside of the test object.

An ultrasonic wave, a photoacoustic wave, or the like is appropriately selected as the acoustic wave according to the test object or measurement conditions.

For example, an ultrasound diagnostic apparatus, which is a kind of the acoustic wave measurement apparatus, transmits an ultrasonic wave to the inside of the test object, receives the ultrasonic wave reflected by tissues inside the test object, and displays the received ultrasonic wave as an image.

In addition, a photoacoustic wave measurement apparatus, which is a kind of the acoustic wave measurement apparatus, receives an acoustic wave radiated from the inside of the test object due to a photoacoustic effect, and displays the received acoustic wave as an image. The photoacoustic effect is a phenomenon in which an acoustic wave (typically, an ultrasonic wave) is generated through thermal expansion after the test object absorbs an electromagnetic wave to generate heat in a case where the test object is irradiated with an electromagnetic wave pulse of visible light, near infrared light, microwave, or the like.

Since the acoustic wave measurement apparatus transmits and receives an acoustic wave to and from the test object, the acoustic wave probe is required to match an acoustic impedance with the test object (typically, a human body). In order to satisfy this requirement, the acoustic wave probe is provided with an acoustic matching layer. This will be described by taking, as an example, a probe for an ultrasound diagnostic apparatus (also referred to as an ultrasound probe), which is a kind of the acoustic wave probe.

The ultrasound probe includes a piezoelectric element that transmits and receives an ultrasonic wave and an acoustic lens which comes into contact with a living body, in which an acoustic matching layer is disposed between the piezoelectric element and the acoustic lens. An ultrasonic wave oscillated from the piezoelectric element is incident on the living body after being transmitted through the acoustic matching layer, further being transmitted through the acoustic lens. There is usually a difference in acoustic impedance (density × longitudinal wave acoustic velocity) between the acoustic lens and the living body. In a case where this difference is large, the ultrasonic wave is easily reflected on a surface of the living body, and an incident efficiency of the ultrasonic wave into the living body is lowered. Therefore, the acoustic lens is required to have an acoustic impedance characteristic close to that of the living body.

On the other hand, the difference in acoustic impedance between the piezoelectric element and the living body is generally large. Accordingly, the difference in acoustic impedance between the piezoelectric element and the acoustic lens is also usually large. Therefore, in a case of a laminated structure of the piezoelectric element and the acoustic lens, the ultrasonic wave emitted from the piezoelectric element is reflected on a surface of the acoustic lens, and the incident efficiency of the ultrasonic wave into the living body is lowered. In order to suppress this reflection of the ultrasonic wave, the above-described acoustic matching layer is provided between the piezoelectric element and the acoustic lens. The acoustic impedance of the acoustic matching layer takes a value between the acoustic impedance of the living body or the acoustic lens and the acoustic impedance of the piezoelectric element, which leads to improved propagation efficiency of an ultrasonic wave from the piezoelectric element to the living body. In addition, in recent years, the development of an acoustic matching layer with more efficient propagation of acoustic impedance of an ultrasonic wave has been underway by providing a gradient in acoustic impedance from the acoustic lens side to the piezoelectric element side, that is, by increasing the acoustic impedance of the acoustic matching layer as it approaches the piezoelectric element from the acoustic lens, through a configuration of an acoustic matching layer having a multi-layer structure in which a plurality of acoustic matching sheets (sheet-like acoustic matching layer materials) are laminated.

For example, JP2009-296055A discloses a technique in which, in order to realize an acoustic matching sheet having a desired acoustic impedance, an acoustic matching layer (acoustic matching sheet) using an epoxy resin with silica particles, glass particles, and metal particles (for example, tungsten, zinc, aluminum, tin, silver, platinum, and gold) is obtained.

### SUMMARY OF THE INVENTION

The acoustic matching layer is required to have higher impedance toward the piezoelectric element side, and an acoustic impedance of 10 Mrayl or more is required from the interlayer to the piezoelectric element side with respect to a thickness direction of the acoustic matching layer. In a case where high-density particles such as metal particles are simply used to increase the acoustic impedance of the acoustic matching sheet, the longitudinal wave acoustic velocity is lowered, and as a result, there are restrictions on realization of the high acoustic impedance. As a result of studies by the present inventors, it has been found that, by using a certain amount of low-density particles with respect to high-density metal particles, a decrease in longitudinal wave acoustic velocity of the acoustic matching sheet can be suppressed while taking the effect of high density. However, in a case where the high-density metal particles and the low-density particles are used in combination, it has been found that air bubbles are likely to be entrained during mixing of raw materials in the manufacturing process, and remain in the acoustic matching sheet to be obtained, which tends to reduce yield. In addition, it has been found that the acoustic matching sheet to be obtained has deteriorated mechanical strength due to effects of the remaining air bubbles or low-density particles, and tends to deteriorate in workability such as cutting.

An object of the present invention is to provide, using metal particles having a density in a specific range, an acoustic matching layer material which exhibits high acoustic impedance, has low air bubble content, and has sufficient mechanical strength; an acoustic matching sheet formed of the acoustic matching layer material; and a composition for an acoustic matching layer material, suitable for preparing the acoustic matching layer material.

Another object of the present invention is to provide an acoustic wave probe using the acoustic matching sheet, and an acoustic wave measurement apparatus using the acoustic wave probe.

Another object of the present invention is to provide a manufacturing method of an acoustic wave probe using the acoustic matching layer material.

As a result of further studies to obtain an acoustic matching layer material which exhibits high acoustic impedance and is suitable for an acoustic matching layer on a piezoelectric element side, the present inventors have found that, by using, as raw materials of the acoustic matching layer material, an epoxy resin having an epoxy equivalent weight of 140 or less, metal particles, and a specific curing agent in combination, it is possible to effectively suppress the decrease in longitudinal wave acoustic velocity and achieve sufficiently high acoustic impedance while taking the advantage of high density of the material due to the metal particles, and the acoustic matching layer material to be obtained has low air bubble content and sufficient mechanical strength. The present invention has been completed by further repeating studies on the basis of the above-described finding.

That is, the foregoing objects of the present invention have been achieved by the following methods.
<1> An acoustic matching layer material comprising:
   a curing reaction product obtained by reacting an epoxy resin (A) having an epoxy equivalent weight of 140 or less with at least one of a curing agent (B-1) which is a monomer or a curing catalyst (B-2); and
   metal particles (C) having a density of 4.5 g/cm³ or more and less than 10 g/cm³ at 20°C,
   in which a content of particles (D) having a density of less than 4.5 g/cm³ at 20°C is less than 5% by mass.
<2> The acoustic matching layer material according to <1>,
   in which the epoxy resin (A) is a compound represented by any one of General Formula (1), ..., or (4), 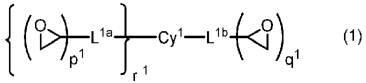
   in General Formula (1), Cy¹ represents a ring, L^{1a} represents a linking group and L^{1b} represents a linking group containing a nitrogen atom, p¹ is 1 or 2, q¹ is 1 or 2, and r¹ is an integer of 1 to 3, 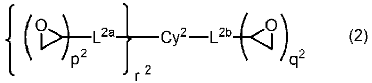 in General Formula (2), Cy² represents a ring, L^{2a} and L^{2b} represent an alkylene group, an alkanetriyl group, an oxygen atom, or a linking group obtained by combining these groups, p² is 1 or 2, q² is 1 or 2, and r² is an integer of 1 to 3, 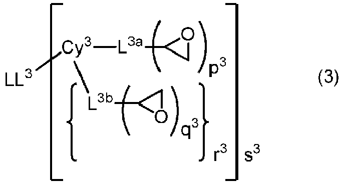
      in General Formula (3), Cy³ represents a ring, L^{3a} represents a linking group containing a nitrogen atom and L^{3b} represents a linking group, LL³ represents a linking group, p³ is 1 or 2, q³ is 1 or 2, r³ is an integer of 0 to 3, and s³ is 2 or 3,
      where the compound represented by General Formula (3) has three or more epoxy groups, 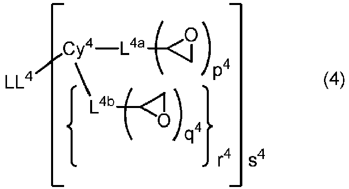
      in General Formula (4), Cy⁴ represents a ring, L^{4a} and L^{4b} represent an alkylene group, an alkanetriyl group, an oxygen atom, or a linking group obtained by combining these groups, LL⁴ represents a linking group, p⁴ is 1 or 2, q⁴ is 1 or 2, r⁴ is an integer of 0 to 3, and s⁴ is 2 or 3,
      where the compound represented by General Formula (4) has three or more epoxy groups.
<3> The acoustic matching layer material according to <1> or <2>,
   in which the curing agent (B-1) includes an amine compound.
<4> The acoustic matching layer material according to any one of <1> to <3>,
   in which the curing agent (B-1) includes a primary amine compound.
<5> The acoustic matching layer material according to any one of <1> to <4>,
   in which the curing agent (B-1) includes an aromatic ring-containing primary amine compound.
<6> The acoustic matching layer material according to any one of <1> to <5>,
   in which the epoxy resin (A) has an aromatic hydrocarbon ring.
<7> The acoustic matching layer material according to any one of <1> to <6>,
   in which a longitudinal wave acoustic velocity of an ultrasonic wave at 25°C is 2,700 m/sec or more.
<8> The acoustic matching layer material according to any one of <1> to <7>,
   in which an acoustic impedance at 25°C is 12 Mrayl or more.
<9> An acoustic matching sheet consisting of the acoustic matching layer material according to any one of <1> to <8>.
<10> A composition for an acoustic matching layer material, which is used for obtaining the acoustic matching layer material according to any one of <1> to <8>, the composition comprising:
   an epoxy resin (A) having an epoxy equivalent weight of 140 or less;
   at least one of a curing agent (B-1) which is a monomer or a curing catalyst (B-2); and
   metal particles (C) having a density of 4.5 g/cm³ or more and less than 10 g/cm³ at 20°C,
   in which a content of particles (D) having a density of less than 4.5 g/cm³ at 20°C in a solid content is less than 5% by mass.
<11> An acoustic wave probe comprising:
   the acoustic matching sheet according to <9> as an acoustic matching layer.
<12> An acoustic wave measurement apparatus comprising:
   the acoustic wave probe according to <11>.
<13> The acoustic wave measurement apparatus according to <12>,
   in which the acoustic wave measurement apparatus is an ultrasound diagnostic apparatus.
<14> A manufacturing method of an acoustic wave probe, comprising:
   forming an acoustic matching layer on a piezoelectric element using the acoustic matching layer material according to any one of <1> to <8>.

The expression "to" in the present specification is used to mean that numerical values described before and after "to" are included as a lower limit value and an upper limit value, respectively.

The "epoxy equivalent weight" in the present specification is the number of grams (g/eq) of an epoxy resin containing 1-gram equivalent epoxy group. That is, the "epoxy equivalent" refers to a value obtained by dividing a molecular weight of the epoxy resin by the number of epoxy groups contained in the epoxy resin.

In the present specification, in a case of a plurality of substituents, linking groups, and the like (hereinafter, referred to as a substituent and the like) represented by a specific reference numeral, or in a case of simultaneously or alternatively defining a plurality of the substituent and the like, it means that each of the substituent and the like may be the same or different from each other. In addition, unless specified otherwise, in a case where a plurality of substituents or the like are adjacent to each other, the substituents may be linked or fused to each other to form a ring.

In the present specification, the term "group" for each of the groups described as examples of each of substituents is used to mean to include both an unsubstituted form and a form having a substituent. For example, "alkyl group" means an alkyl group which may have a substituent. In addition, in a case where the number of carbon atoms of the group is limited, the number of carbon atoms of the group means the total number of carbon atoms including a substituent, unless otherwise specified.

In the present specification, the expression of a compound is used to include the compound itself, a salt thereof, and an ion thereof. In addition, it is meant to also include compounds in which a part of the structure is changed, as long as the effects of the present invention are not impaired. Furthermore, a compound which is not specifically described as substituted or unsubstituted may have an optional substituent within a range which does not impair the effects of the present invention. The same applies to a substituent and a linking group.

By using metal particles having a density in a specific range, the acoustic matching layer material according to the aspect of the present invention and the acoustic matching sheet formed of the acoustic matching layer material exhibit high acoustic impedance, have low air bubble content, and have sufficient mechanical strength.

In addition, by curing the composition for an acoustic matching layer material according to the aspect of the present invention, the above-described acoustic matching sheet can be obtained.

In addition, the acoustic wave probe according to the aspect of the present invention includes the above-described acoustic matching sheet.

In addition, the acoustic wave measurement apparatus according to the aspect of the present invention includes the acoustic wave probe.

In addition, with the manufacturing method of the acoustic wave probe according to the aspect of the present invention, an acoustic wave probe using the above-described acoustic matching layer material can be obtained.

### BRIEF DESCRIPTION OF THE DRAWINGS

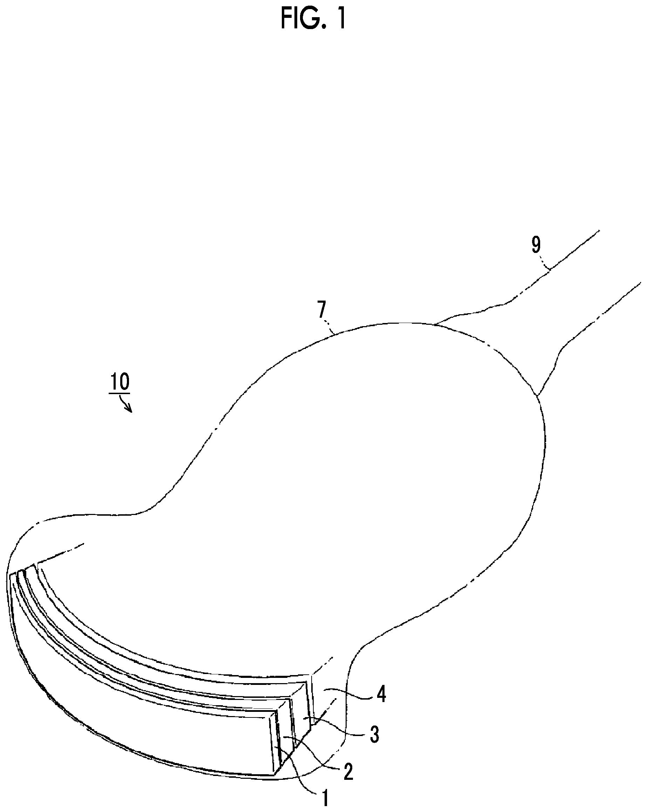

Fig. 1 is a perspective view of an example of a convex type ultrasound probe which is an aspect of an acoustic wave probe.

### DESCRIPTION OF THE PREFERRED EMBODIMENTS

### [Acoustic matching layer material]

The acoustic matching layer material according to the embodiment of the present invention (hereinafter, also simply referred to as "layer material according to the embodiment of the present invention") contains a curing reaction product obtained by reacting an epoxy resin (A) having an epoxy equivalent weight of 140 or less with at least one of a curing agent (B-1) which is a monomer or a curing catalyst (B-2), and metal particles (C) having a density of 4.5 g/cm³ or more and less than 10 g/cm³ at 20°C.

The layer material according to the embodiment of the present invention may contain particles (D) having a density of less than 4.5 g/cm³ at 20°C, and in the layer material according to the embodiment of the present invention, a content of the particles (D) is less than 5% by mass.

Hereinafter, the "epoxy resin (A) having an epoxy equivalent weight of 140 or less" may be simply referred to as "epoxy resin (A)". The "curing agent (B-1) which is a monomer" may be simply referred to as "curing agent (B-1)". The "metal particles (C) having a density of 4.5 g/cm³ or more and less than 10 g/cm³ at 20°C" may be simply referred to as "metal particles (C)". The "particles (D) having a density of less than 4.5 g/cm³ at 20°C" may be simply referred to as "particles (D)".

A shape of the layer material according to the embodiment of the present invention is not particularly limited, and examples thereof include a sheet shape, a columnar shape, and a prismatic shape, and a sheet shape is preferable.

The layer material according to the embodiment of the present invention has, as a binding material, the curing reaction product obtained by reacting the epoxy resin (A) with at least one of the curing agent (B-1) or the curing catalyst (B-2). Therefore, the metal particles (C) are contained in the binding material (matrix) having a large elastic modulus, and it is considered that this is responsible for increasing the speed of longitudinal wave acoustic velocity and ultimately achieving high acoustic impedance. In addition, since high acoustic impedance can be achieved even without the low-density particles (D), it is considered that air bubbles are less likely to be entrained during mixing of raw materials in the production, which is related to sufficient mechanical strength.

Hereinafter, the curing reaction product obtained by reacting the epoxy resin (A) with at least one of the curing agent (B-1) which is a monomer or the curing catalyst (B-2) may be referred to as "binder".

### (Epoxy resin (A))

The epoxy resin (A) contained in the layer material according to the embodiment of the present invention is not particularly limited as long as it is an epoxy resin having an epoxy equivalent weight of 140 or less.

The lower limit of the epoxy equivalent weight of the epoxy resin (A) is not particularly limited, and is, for example, 60 or more, preferably 70 or more.

A molecular weight of the epoxy resin (A) is not particularly limited, and is, for example, 150 to 800, preferably 200 to 700. The number of epoxy groups per molecule of the epoxy resin (A) is not particularly limited, and is, for example, 2 to 10 and may be 2 to 8.

The epoxy resin (A) is preferably a compound represented by any one of General Formula (1), ..., (4), and from the reason that, by curing quickly, it is possible to simultaneously increase the speed of acoustic velocity and the acoustic impedance, a compound represented by General Formula (1) is more preferable. In addition, from the viewpoint of high toughness of the acoustic matching layer material, the epoxy resin (A) preferably has an aromatic hydrocarbon ring.

-Compound represented by General Formula (1)-

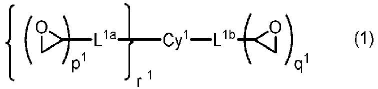

In General Formula (1), Cy¹ represents a ring, L^{1a} represents a linking group and L^{1b} represents a linking group containing a nitrogen atom, p¹ is 1 or 2, q¹ is 1 or 2, and r¹ is an integer of 1 to 3.

Cy¹ may be a monocyclic ring or a fused ring.

Examples of Cy¹ include an alicyclic ring, an aliphatic heterocyclic ring, an aromatic hydrocarbon ring, and an aromatic heterocyclic ring, and an alicyclic ring or an aromatic hydrocarbon ring is preferable and an aromatic hydrocarbon ring is more preferable.

The number of ring-constituting carbon atoms in the alicyclic ring is not particularly limited, and is, for example, 3 to 10, preferably 5 to 8 and more preferably 6. Specific examples of the alicyclic ring include a cyclohexane ring.

The number of ring-constituting atoms in the aliphatic heterocyclic ring is not particularly limited, and is, for example, 6 to 10, preferably 6. Examples of a ring-constituting heteroatom of the aromatic heterocyclic ring include a nitrogen atom and an oxygen atom.

The number of ring-constituting carbon atoms in the aromatic hydrocarbon ring is not particularly limited, and is, for example, 6 to 10. Specific examples of the aromatic hydrocarbon ring include a benzene ring and a naphthalene ring.

The number of ring-constituting atoms in the aromatic heterocyclic ring is not particularly limited, and is, for example, 6 to 10. Examples of a ring-constituting heteroatom of the aromatic heterocyclic ring include a nitrogen atom and an oxygen atom. Specific examples of the aromatic heterocyclic ring include a pyridine ring.

Cy¹ may have a substituent, and specific examples of the substituent include an alkyl group (for example, having 1 to 5 carbon atoms), an oxo group, an alkoxy group (for example, having 1 to 5 carbon atoms), an amino group, an aryl group (for example, a phenyl group and a naphthyl group), and a halogen atom (for example, a fluorine atom, a chlorine atom, a bromine atom, and an iodine atom).

As the linking group which can be adopted as L^{1a}, an alkylene group, an alkanetriyl group, a nitrogen atom, an oxygen atom, or a linking group obtained by combining these groups is preferable.

The alkylene group may be linear or branched, and the number of carbon atoms in the alkylene group is, for example, 1 to 10, preferably 1 to 5, more preferably 1 or 2, and particularly preferably 1. Specific examples of the alkylene group include methylene, ethylene, propylene, and isopropylene.

The alkanetriyl group may be linear or branched, and the number of carbon atoms in the alkanetriyl group is, for example, 1 to 10, preferably 1 to 6 and more preferably 1 to 4. Specific examples of the alkanetriyl group include methanetriyl, ethanetriyl, and propanetriyl.

Examples of the "linking group obtained by combining these groups" described above include a divalent linking group obtained by combining an alkylene group and an oxygen atom ("-alkylene-O-" and "-O-alkylene-"), and a trivalent linking group obtained by combining an alkylene group and a nitrogen atom ("(-alkylene-)₂nitrogen atom-", "-nitrogen atom(-alkylene-)₂", "(-alkylene-)₂nitrogen atom-alkylene-", and "-alkylene-nitrogen atom(-alkylene-)₂").

Examples of the linking group which can be adopted as L^{1b} include a divalent linking group obtained by combining an imino group and an alkylene group ("-NH-alkylene group-" and "-alkylene group-NH-"), a trivalent group obtained by combining an alkylene group and a nitrogen atom ("(-alkylene-)₂nitrogen atom-", "-nitrogen atom(-alkylene-)₂", "(-alkylene-)₂nitrogen atom-alkylene-", and "-alkylene-nitrogen atom(-alkylene-)₂"). A preferred aspect of the alkylene group is the same as the aspect of the alkylene group described in L^{1a}.

Specific examples of the compound represented by General Formula (1) are shown below, but the present invention is not limited thereto. In the following, "Mw" means a molecular weight, and "EEW" means an epoxy equivalent weight. The same applies to specific examples of the compound represented by any one of General Formula (2), (3), or (4) described below.

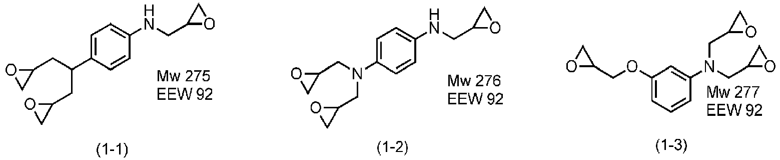

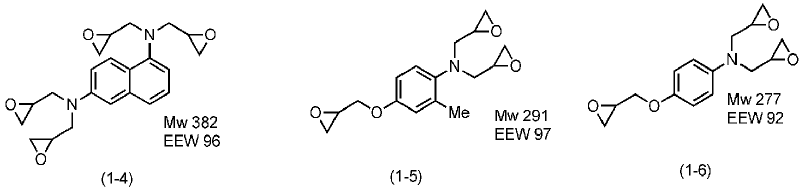

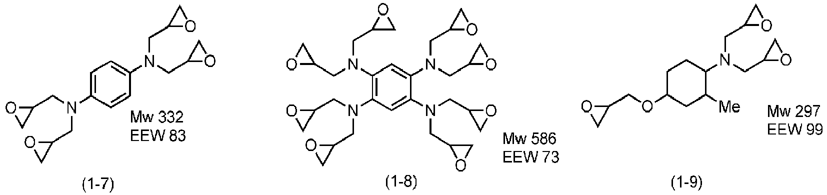

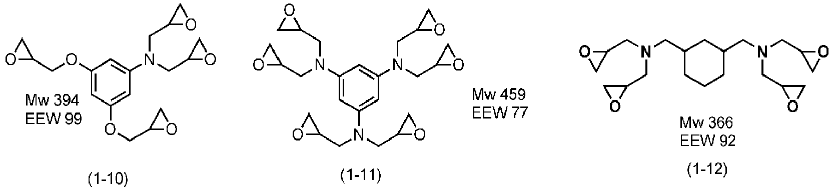

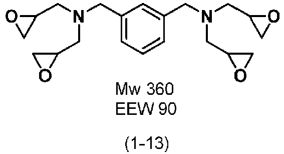

-Compound represented by General Formula (2)-

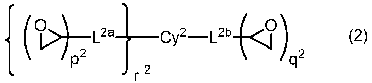

In General Formula (2), Cy² represents a ring, L^{2a} and L^{2b} represent an alkylene group, an alkanetriyl group, an oxygen atom, or a linking group obtained by combining these groups, p² is 1 or 2, q² is 1 or 2, and r² is an integer of 1 to 3 (preferably 1 or 2).

Cy² may be a monocyclic ring or a fused ring.

Examples of Cy² include an alicyclic ring, an aliphatic heterocyclic ring, an aromatic hydrocarbon ring, and an aromatic heterocyclic ring, and an aromatic hydrocarbon ring is preferable.

Examples of the alicyclic ring, the aliphatic heterocyclic ring, the aromatic hydrocarbon ring, and the aromatic heterocyclic ring, which can be adopted as Cy², include the alicyclic ring, the aliphatic heterocyclic ring, the aromatic hydrocarbon ring, and the aromatic heterocyclic ring described in "Cy¹" above.

Cy² may have a substituent, and specific examples of the substituent include the substituents described in "Cy¹" above.

The alkylene group may be linear or branched, and the number of carbon atoms in the alkylene group is, for example, 1 to 10, preferably 1 to 5, more preferably 1 or 2, and particularly preferably 1. Specific examples of the alkylene group include methylene, ethylene, propylene, and isopropylene.

The alkanetriyl group may be linear or branched, and the number of carbon atoms in the alkanetriyl group is, for example, 1 to 10, preferably 1 to 6 and more preferably 1 to 4. Specific examples of the alkanetriyl group include methanetriyl, ethanetriyl, and propanetriyl.

Examples of the "divalent or trivalent linking group obtained by these groups" include a divalent group obtained by combining an alkylene group and an oxygen atom ("-alkylene-O-" and "-O-alkylene-").

Specific examples of the compound represented by General Formula (2) are shown below, but the present invention is not limited thereto.

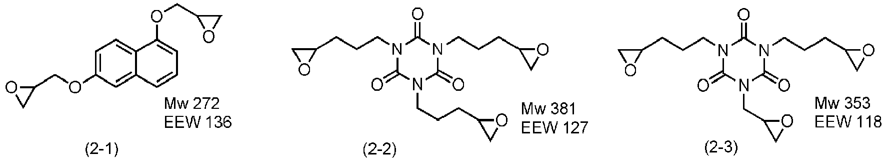

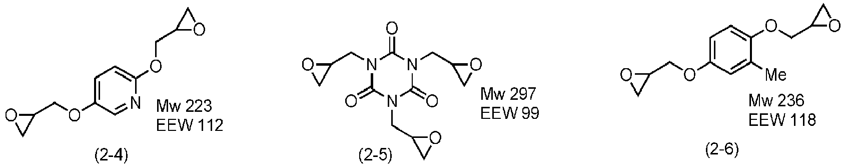

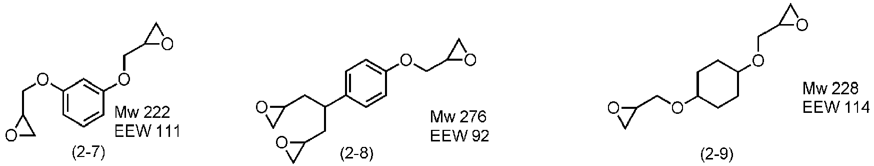

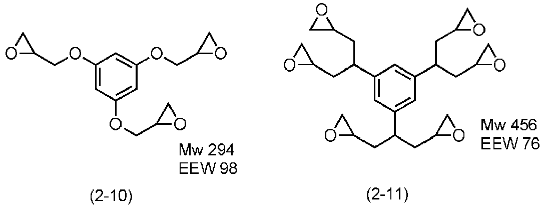

-Compound represented by General Formula (3)-

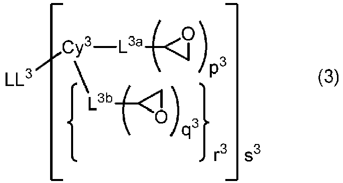

In General Formula (3), Cy³ represents a ring, L^{3a} represents a linking group containing a nitrogen atom and L^{3b} represents a linking group, LL³ represents a linking group, p³ is 1 or 2, q³ is 1 or 2, r³ is an integer of 0 to 3 (preferably 0 or 1), and s³ is 2 or 3,
where the compound represented by General Formula (3) has three or more epoxy groups.

Cy³ may be a monocyclic ring or a fused ring.

Examples of Cy³ include an alicyclic ring, an aliphatic heterocyclic ring, an aromatic hydrocarbon ring, and an aromatic heterocyclic ring, and an aromatic hydrocarbon ring is preferable.

Examples of the alicyclic ring, the aliphatic heterocyclic ring, the aromatic hydrocarbon ring, and the aromatic heterocyclic ring, which can be adopted as Cy³, include the alicyclic ring, the aliphatic heterocyclic ring, the aromatic hydrocarbon ring, and the aromatic heterocyclic ring described in "Cy¹" above, and an aromatic hydrocarbon ring is preferable.

Cy³ may have a substituent, and specific examples of the substituent include the substituents described in "Cy¹" above.

Examples of the linking group which can be adopted as L^{3a} include a trivalent group obtained by combining an alkylene group and a nitrogen atom ("(-alkylene-)₂nitrogen atom-" and "-nitrogen atom(-alkylene-)₂").

As the linking group which can be adopted as L^{3b}, an alkylene group, a nitrogen atom, an oxygen atom, or a linking group obtained by combining these groups is preferable.

The alkylene group may be linear or branched, and the number of carbon atoms in the alkylene group is, for example, 1 to 10, preferably 1 to 5, more preferably 1 or 2, and particularly preferably 1. Specific examples of the alkylene group include methylene, ethylene, propylene, and isopropylene.

Examples of the "linking group obtained by combining these groups" described above include a divalent linking group obtained by combining an alkylene group and an oxygen atom ("-alkylene-O-" and "-O-alkylene-"), and a trivalent linking group obtained by combining an alkylene group and a nitrogen atom ("(-alkylene-)₂nitrogen atom-" and "-nitrogen atom(-alkylene-)₂").

As the linking group which can be adopted as L^{3b}, the "divalent linking group obtained by combining an alkylene group and an oxygen atom" described above or the "trivalent linking group obtained by combining an alkylene group and a nitrogen atom" described above is preferable.

Examples of the divalent linking group which can be adopted as LL³ include an alkylene group and a sulfonyl group.

The alkylene group may be linear or branched, and the number of carbon atoms in the alkylene group is, for example, 1 to 10, preferably 1 to 5, more preferably 1 or 2, and particularly preferably 1. Specific examples of the alkylene group include methylene, ethylene, propylene, and isopropylene.

Examples of the trivalent linking group which can be adopted as LL³ include an alkanetriyl group.

The alkanetriyl group may be linear or branched, and the number of carbon atoms in the alkanetriyl group is, for example, 1 to 10, preferably 1 to 5, more preferably 1 or 2, and particularly preferably 1. Specific examples of the alkanetriyl group include methanetriyl, ethanetriyl, and propanetriyl.

Specific examples of the compound represented by General Formula (3) are shown below, but the present invention is not limited thereto.

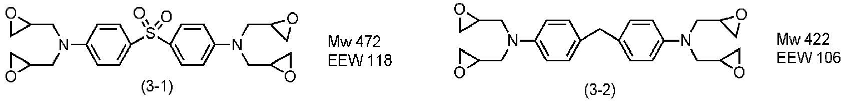

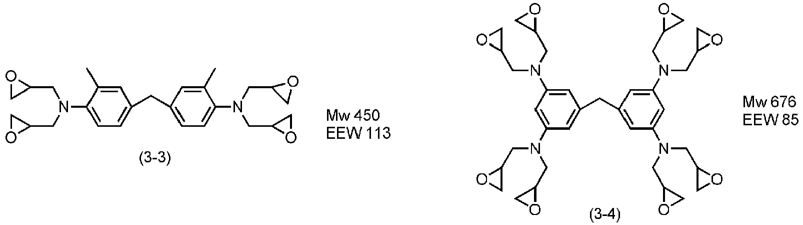

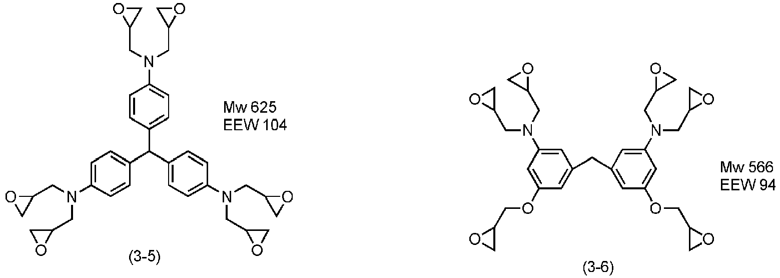

-Compound represented by General Formula (4)-

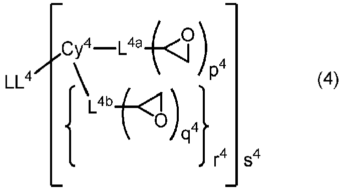

In General Formula (4), Cy⁴ represents a ring, L^{4a} and L^{4b} represent an alkylene group, an alkanetriyl group, an oxygen atom, or a linking group obtained by combining these groups, LL⁴ represents a linking group, p⁴ is 1 or 2, q⁴ is 1 or 2, r⁴ is an integer of 0 to 3 (preferably 1), and s⁴ is 2 or 3 (preferably 2),
where the compound represented by General Formula (4) has three or more epoxy groups.

Cy⁴ may be a monocyclic ring or a fused ring.

Examples of Cy⁴ include an alicyclic ring, an aliphatic heterocyclic ring, an aromatic hydrocarbon ring, and an aromatic heterocyclic ring, and an aromatic hydrocarbon ring is preferable.

Examples of the alicyclic ring, the aliphatic heterocyclic ring, the aromatic hydrocarbon ring, and the aromatic heterocyclic ring, which can be adopted as Cy⁴, include the alicyclic ring, the aliphatic heterocyclic ring, the aromatic hydrocarbon ring, and the aromatic heterocyclic ring described in "Cy¹" above, and an aromatic hydrocarbon ring is preferable.

Cy⁴ may have a substituent, and specific examples of the substituent include the substituents described in "Cy¹" above.

The alkylene group which can be adopted as L^{4a} and L^{4b} may be linear or branched, and the number of carbon atoms in the alkylene group is, for example, 1 to 10, and may be 1 to 5 or 1 or 2. Specific examples of the alkylene group include methylene, ethylene, propylene, and isopropylene.

The alkanetriyl group may be linear or branched, and the number of carbon atoms in the alkanetriyl group is, for example, 1 to 10, and may be 1 to 6 or 1 to 4. Specific examples of the alkanetriyl group include methanetriyl, ethanetriyl, and propanetriyl.

Examples of the "linking group obtained by these groups" include a divalent group obtained by combining an alkylene group and an oxygen atom ("-alkylene-O-" and "-O-alkylene-").

Examples of the divalent linking group which can be adopted as LL⁴ include an alkylene group.

The alkylene group may be linear or branched, and the number of carbon atoms in the alkylene group is, for example, 1 to 10, preferably 1 to 5 and preferably 1 to 3. Specific examples of the alkylene group include methylene, ethylene, propylene, and 1-methylethylidene.

Examples of the trivalent linking group which can be adopted as LL⁴ include an alkanetriyl group.

The alkanetriyl group may be linear or branched, and the number of carbon atoms in the alkanetriyl group is, for example, 1 to 10, preferably 1 to 5, more preferably 1 or 2, and particularly preferably 1. Specific examples of the alkanetriyl group include methanetriyl, ethanetriyl, and propanetriyl.

Specific examples of the compound represented by General Formula (4) are shown below, but the present invention is not limited thereto.

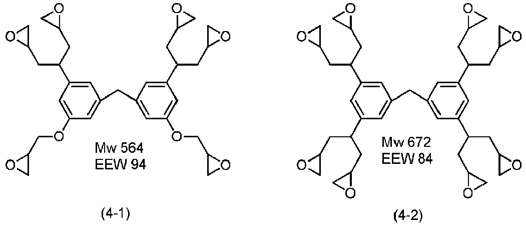

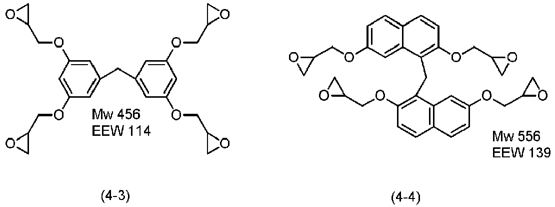

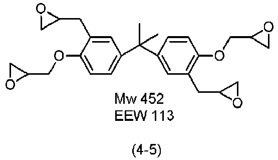

The epoxy resin (A) may be used alone or in combination of two or more thereof.

In the layer material according to the embodiment of the present invention, the curing reaction product which is a binding material may be a product obtained by polymerizing and curing the epoxy resin (A) by action of the curing catalyst (B-2), or may be a product obtained by reacting and curing the epoxy resin (A) with the curing agent (B-1).

### (Curing agent (B-1) and curing catalyst (B-2))

As the curing agent (B-1) used in the present invention, various curing agents generally used as a curing agent for an epoxy resin, which are a monomer, can be used. As the curing agent (B-1), for example, an amine curing agent, an acid anhydride curing agent, a phenol curing agent, a thiol curing agent, dicyandiamide, or the like can be used.

On the other hand, as the curing catalyst (B-2) used in the present invention, various catalysts commonly used as a catalyst for a curing reaction of the epoxy resin can be used. As the curing catalyst (B-2), for example, low-molecular-weight compounds (for example, a molecular weight of 50 to 800) such as an imidazole curing catalyst, a phosphine curing catalyst, a Lewis acid curing catalyst, and an amine curing catalyst can be used.

In the layer material according to the embodiment of the present invention, it is preferable that the curing reaction product includes a curing reaction product of the epoxy resin (A) and the curing agent (B-1). From the viewpoint of curing temperature and curing rate, the curing agent (B-1) is preferably an amine compound. The amine compound is preferably a primary amine compound. In addition, it is also preferably an aromatic ring-containing amine compound (compound having an aromatic hydrocarbon ring and an amino group). Among these, the curing agent (B-1) is particularly preferable an aromatic ring-containing primary amine compound (compound having an aromatic hydrocarbon ring and an unsubstituted amino group).

The aromatic ring contained in the aromatic ring-containing amine compound may be a monocyclic ring or a fused ring, but is preferably a monocyclic ring. The number of aromatic rings contained in the aromatic ring-containing amine compound is not particularly limited, and is preferably 1 or 2 and more preferably 1.

The aromatic ring-containing primary amine compound may have a monosubstituted amino group in addition to the unsubstituted amino group.

In the present invention, a plurality of the curing agents (B-1) and the curing catalysts (B-2) may be used, and it is also preferable to add a small amount of one thereof as a curing aid.

The curing agent (B-1) reacts with the epoxy resin (A) to constitute a curing reaction product.

Specific examples of the curing agent (B-1) and the curing catalyst (B-2) are shown below, but the present invention is not limited thereto. The following "Equivalent" means an active hydrogen equivalent.

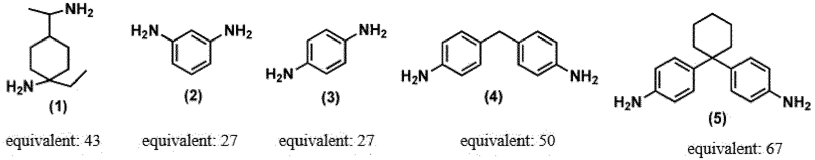

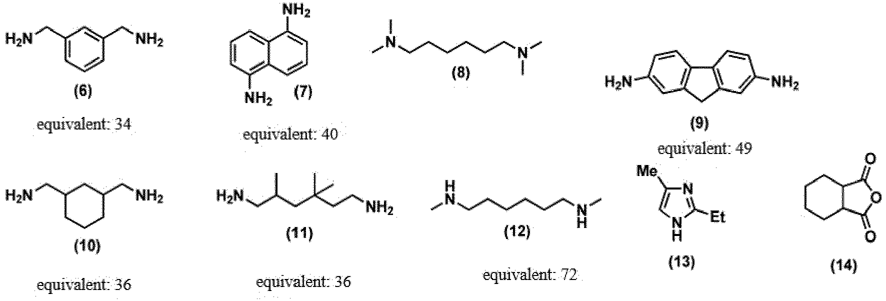

### (Metal particles (C))

The layer material according to the embodiment of the present invention contains the metal particles (C). By adjusting a content of the metal particles (C) in the layer material, a density of the layer material can be adjusted, and an acoustic impedance of the layer material can be adjusted to a desired level. The metal particles (C) may or may not be surface-treated. The surface treatment can be performed, for example, with reference to WO2019/088148A.

A metal constituting the metal particles (C) is not particularly limited as long as the density is 4.5 g/cm³ or more and less than 10 g/cm³. The above-described density may be 4.8 g/cm³ or more and less than 10 g/cm³. The metal particles (C) may be a metal atom alone, or may be a carbide, a nitride, an oxide or a boride of the metal. In addition, an alloy may be formed.

Examples of a metal constituting the metal particles include copper, nickel, cobalt, niobium, iron, manganese, tin, zinc, zirconium, vanadium, zirconium, and titanium. In particular, copper, iron, zinc, zirconium, or titanium is preferable, and it is also preferable to use carbides, oxides, or nitrides thereof. Copper, iron, or titanium is more preferable, and it is also preferable to use carbides, oxides, or nitrides thereof.

A particle size of the metal particles (C) is not particularly limited. From the viewpoint of reducing the viscosity of the composition for an acoustic matching layer material and improving the mechanical strength of the acoustic matching layer material, the particle size of the metal particles (C) is, for example, preferably 0.01 to 100 µm, more preferably 1 to 10 µm, still more preferably 2 to 6 µm, and particularly preferably 2 to 4 µm.

The "particle size" of the metal particles (C) means an average primary particle diameter. Here, the average primary particle diameter is a volume-based median diameter, and is determined as follows.

The metal particles (C) are added to methanol in an amount of 0.5% by mass and subjected to ultrasonic wave for 10 minutes to disperse the metal particles (C). A particle size distribution of the metal particles (C) treated as described above is measured with a laser diffraction scattering-type particle size distribution analyzer (manufactured by HORIBA, Ltd., trade name: LA950V2), thereby determining the volume-based median diameter. The median diameter corresponds to a cumulative 50% in a case where the particle size distribution is represented as a cumulative distribution.

### (Particles (D))

The particles (D) are not particularly limited as long as they are particles having a density of 4.5 g/cm³ or less. As the particles (D), metal particles, ceramic particles, organic fine particles, silica particles, or organic-inorganic composite particles can be used.

As a metal constituting the metal particles, for example, barium, aluminum, boron, oxides thereof, nitrides thereof, or carbides thereof can be used.

The ceramic particles preferably contain at least one atom of periodic table Groups 1 to 3 or 13 to 17, and are more preferably a substance containing at least one (preferably, one to three) of Mg, Ca, Ba, B, Al, Y, or Si and at least one (preferably, one) of O, C, N, or S.

As the ceramic particles, carbides, nitrides, or oxides containing at least one (preferably, one to three) of Mg, Ba, B, Al, Y, or Si are preferable, and specific examples thereof include magnesium-aluminum spinel (magnesium aluminate spinel, MgO·Al₂O₃), wollastonite (CaSiO₃), cordierite (2MgO·2Al₂O₃·5SiO₂), boron carbide (B₄C), silicon carbide (SiC), alumina (Al₂O₃), aluminum nitride (AlN), magnesium oxide (MgO), silicon nitride (Si₃N₄), boron nitride (BN), and yttrium oxide (Y₂O₃).

As the organic fine particles, rubber particles, acrylic particles, melamine particles, carbon black, or graphite can be used.

As the silica particles, fumed silica or fused silica can be used.

As the organic-inorganic composite particles, silicone acrylic particles can be used.

A particle size of the particles (D) is not particularly limited. From the viewpoint of reducing the viscosity of the composition for an acoustic matching layer material and improving the mechanical strength of the acoustic matching layer material, the particle size of the particles (D) is, for example, preferably 0.01 to 100 µm, more preferably 1 to 10 µm, still more preferably 2 to 6 µm, and particularly preferably 2 to 4 µm.

The "particle size" of the particles (D) has the same meaning as the "particle size" of the metal particles (C).

In the layer material according to the embodiment of the present invention, respective contents of the binding material, the metal particles (C), and the particles (D) are appropriately adjusted according to a desired longitudinal wave acoustic velocity and acoustic impedance.

The content of the binding material in the layer material according to the embodiment of the present invention is preferably 1% to 15% by mass and more preferably 1% to 11% by mass. The content of the metal particles (C) in the layer material according to the embodiment of the present invention is preferably 80% to 98% by mass, more preferably 85% to 95% by mass, and still more preferably 86% to 92% by mass. The content of the particles (D) in the layer material according to the embodiment of the present invention is 5% by mass or less, more preferably 2% by mass or less, still more preferably less than 1% by mass, and particularly preferably 0.8% by mass or less.

The layer material according to the embodiment of the present invention may be composed of the binding material and the metal particles (C), or the binding material, the metal particles (C), and the particles (D). In addition, a component other than the binding material and the inorganic filler particles may be contained as long as the effects of the present invention are not impaired. In addition to the binding material, examples of a component other than the metal particles (C) and the particles (D) (other components) include a curing retarder, a dispersant, a pigment, a dye, an antistatic agent, an antioxidant, a flame retardant, and a thermal conductivity improver.

In the layer material according to the embodiment of the present invention, the total content of the binding material, the metal particles (C), and the particles (D) is preferably 80% by mass or more and more preferably 90% by mass or more.

For example, in a case where the layer material according to the embodiment of the present invention is formed into a sheet, an in-plane longitudinal wave acoustic velocity (m/sec) at 25°C is preferably 2,700 or more, more preferably 2,800 or more, and particularly preferably 2,900 or more. The above-described longitudinal wave acoustic velocity is usually 3,200 or less.

In addition, for example, in a case where the layer material according to the embodiment of the present invention is formed into a sheet, an in-plane acoustic impedance (Mrayl) at 25°C is preferably 10 or more, more preferably 12 or more, and particularly preferably 13 or more. The above-described acoustic impedance is usually 15.5 or less.

The longitudinal wave acoustic velocity and the acoustic impedance described above are determined according to the methods described in Examples, which will be described later. Specifically, a layer material processed into a sheet shape is divided into three equal parts in a thickness direction, and with regard to one sheet in the middle of the obtained three sheets, the longitudinal wave acoustic velocity and the acoustic impedance are determined by measuring longitudinal wave acoustic velocity and acoustic impedance at three independent locations. A thickness of the sheet does not substantially affect the longitudinal wave acoustic velocity and the density.

### <Composition for acoustic matching layer material>

The composition for an acoustic matching layer material according to the embodiment of the present invention (a composition which is used in the acoustic matching layer material according to the embodiment of the present invention; hereinafter, also referred to as "composition according to the embodiment of the present invention") contains the epoxy resin (A), at least one of the curing agent (B-1) which is a monomer or the curing catalyst (B-2), and the metal particles (C). The composition according to the embodiment of the present invention may contain the particles (D), and a content of the particles (D) in a solid content contained in the composition according to the embodiment of the present invention is less than 5% by mass. The solid content typically means a component other than a solvent.

In addition, the composition according to the embodiment of the present invention may contain other components described above.

In the composition according to the embodiment of the present invention, a curing reaction of the epoxy resin (A) may progress over time. Therefore, properties of the composition may change with time and may not be stable. However, for example, by storing the above-described composition at a temperature of -10°C or lower, it is possible to obtain a composition in a state in which each component is stably maintained without causing the curing reaction or by sufficiently suppressing the curing reaction.

In addition, it is also preferable to be an aspect of a material set for an acoustic matching layer, in which a resin composition containing the epoxy resin (A) and the metal particles (C) is used as a main agent, and the main agent and at least one of the curing agent (B-1) or the curing catalyst (B-2) are separated in different forms. In preparation of the acoustic matching layer material, the acoustic matching layer material can be prepared by mixing the main agent and at least one of the curing agent (B-1) or the curing catalyst (B-2) to prepare the composition according to the embodiment of the present invention, and then subjecting this composition to a curing reaction.

A mass ratio of the epoxy resin (A), the curing agent (B-1), and the curing catalyst (B-2) constituting the binding material may be appropriately adjusted according to the type of the curing agent (B-1) and the curing catalyst (B-2) used, and the like. For example, the mass of the epoxy resin (A)/the total mass of the curing agent (B-1) and the curing catalyst (B-2) can be set to 99/1 to 20/80, preferably 90/10 to 40/60.

In addition, in a case where the above-described material set for an acoustic matching layer is used for obtaining the composition according to the embodiment of the present invention by mixing the main agent and at least one of the curing agent (B-1) or the curing catalyst (B-2) during the preparation of the layer material, an aspect in which the epoxy resin (A) and at least one of the curing agent (B-1) or the curing catalyst (B-2) are mixed and used in a mass ratio of mass of epoxy resin (A)/total mass of curing agent (B-1) and curing catalyst (B-2) of 99/1 to 20/80 is preferable, and an aspect in which the epoxy resin (A) and at least one of the curing agent (B-1) or the curing catalyst (B-2) are mixed and used in a mass ratio of 90/10 to 40/60 is more preferable.

### <Preparation of composition for acoustic matching layer material>

The composition for an acoustic matching layer material according to the embodiment of the present invention can be obtained, for example, by mixing each component constituting the composition for an acoustic matching layer material. The mixing method is not particularly limited as long as each component can be mixed substantially homogeneously. For example, a desired homogeneous mixing can be achieved by kneading using a rotation and revolution stirrer.

In addition, in a case of a material set for an acoustic matching layer, which contains a main agent consisting of a resin composition containing the epoxy resin (A) and the metal particles (C) and contains at least one of the curing agent (B-1) or the curing catalyst (B-2), the main agent can be obtained by mixing the epoxy resin (A) and the metal particles (C). In preparation of the acoustic matching layer material, the composition for an acoustic matching layer material according to the embodiment of the present invention is obtained by mixing the main agent and at least one of the curing agent (B-1) or the curing catalyst (B-2). The acoustic matching layer material or a precursor thereof can be prepared by curing the composition while molding the composition.

### [Acoustic matching sheet (acoustic matching layer)]

An acoustic matching sheet can be obtained by cutting, dicing, or the like the layer material according to the embodiment of the present invention into a desired thickness or shape, as necessary. In addition, the acoustic matching sheet can be further processed into a desired shape by a conventional method.

Specifically, for example, the composition according to the embodiment of the present invention is shaped into a desired sheet in a low temperature region where a curing reaction does not occur or in a low temperature region where a curing rate is sufficiently slow. Next, the material is heated and cured as necessary to form a crosslinking structure in a molded product, and an acoustic matching sheet or a precursor sheet thereof is obtained by cutting, dicing, or the like into a desired thickness or shape, as necessary. That is, the acoustic matching sheet to be formed is preferably a cured substance obtained by curing the composition according to the embodiment of the present invention to form a three-dimensional network structure. This acoustic matching sheet is used as an acoustic matching layer of an acoustic wave probe. The configuration of the acoustic wave probe including the acoustic matching layer will be described later.

### [Acoustic wave probe]

An acoustic wave probe according to an embodiment of the present invention includes the acoustic matching sheet according to the embodiment of the present invention as at least one layer of an acoustic matching layer.

An example of the configuration of the acoustic wave probe according to the embodiment of the present invention is shown in Fig. 1. The acoustic wave probe shown in Fig. 1 is an ultrasound probe in an ultrasound diagnostic apparatus. The ultrasound probe is a probe which particularly uses an ultrasonic wave as an acoustic wave in an acoustic wave probe. Therefore, a basic structure of the ultrasound probe can be applied to the acoustic wave probe as it is.

### <Ultrasound probe>

An ultrasound probe 10 is a main component of the ultrasound diagnostic apparatus and has a function of generating an ultrasonic wave and transmitting and receiving an ultrasonic beam. As shown in Fig. 1, a configuration of the ultrasound probe 10 is provided in the order of an acoustic lens 1, an acoustic matching layer 2, a piezoelectric element layer 3, and a backing material 4 from a distal end portion (surface coming into contact with a living body which is a test object). In recent years, an ultrasound probe having a laminated structure in which an ultrasonic transducer (piezoelectric element) for transmission and an ultrasonic transducer (piezoelectric element) for reception are formed of materials different from each other has been proposed in order to receive high-order harmonics.

### (Piezoelectric element layer)

The piezoelectric element layer 3 is a portion which generates an ultrasonic wave and in which an electrode is attached to both sides of a piezoelectric element. In a case where voltage is applied to the electrode, the piezoelectric element layer 3 generates an ultrasonic wave through repeated contraction and expansion of the piezoelectric element and through vibration.

A so-called ceramics inorganic piezoelectric body obtained by a polarization treatment of quartz crystals, single crystals such as LiNbO₃, LiTaO₃, and KNbO₃, thin films of ZnO and AlN, Pb(Zr,Ti)O₃-based sintered body, and the like is widely used as the material constituting a piezoelectric element. In general, piezoelectric ceramics such as lead zirconate titanate (PZT) with good conversion efficiency are used.

In addition, sensitivity having a wider band width is required for a piezoelectric element detecting a reception wave on a high frequency side. For this reason, an organic piezoelectric body has been used in which an organic polymer material such as polyvinylidene fluoride (PVDF) is used as the piezoelectric element being suitable for a high frequency or a wide band.

Furthermore, cMUT using micro electro mechanical systems (MEMS) technology in which an array structure, which shows excellent short pulse characteristics, excellent wideband characteristics, and excellent mass productivity and has less characteristic variations, is obtained is described in JP2011-071842A or the like.

In the present invention, it is possible to preferably use any piezoelectric element material.

### (Backing material)

The backing material 4 is provided on a rear surface of the piezoelectric element layer 3 and contributes to the improvement in distance resolution in an ultrasound diagnostic image by shortening the pulse width of an ultrasonic wave through the suppression of excess vibration.

### (Acoustic matching layer)

The acoustic matching layer 2 is provided in order to reduce the difference in acoustic impedance between the piezoelectric element layer 3 and a test object and to efficiently transmit and receive an ultrasonic wave.

### (Acoustic lens)

The acoustic lens 1 is provided to focus an ultrasonic wave in a slice direction by utilizing refraction to improve the resolution. In addition, it is necessary for the acoustic lens 1 to achieve matching of an ultrasonic wave with the acoustic impedance (1.4 to 1.7 Mrayl in a case of a human body) of a living body which is a test object after being closely attached to the living body and to reduce the amount of ultrasonic attenuation of the acoustic lens 1 itself.

That is, by using, as the material of the acoustic lens 1, a material in which the longitudinal wave acoustic velocity is sufficiently lower than the longitudinal wave acoustic velocity of the human body, the attenuation of ultrasonic wave is small, and the acoustic impedance is close to the value of the skin of the human body, sensitivity of transmission and reception of the ultrasonic wave is increased.

The operation of the ultrasound probe 10 having such a configuration will be described. The piezoelectric element layer 3 is resonated after applying a voltage to the electrodes provided on both sides of the piezoelectric element, and an ultrasonic signal is transmitted to a test object from the acoustic lens. During reception of the ultrasonic signal, the piezoelectric element layer 3 is vibrated using the signal (echo signal) reflected from the test object and this vibration is electrically converted into a signal to obtain an image.

### [Manufacturing of acoustic wave probe]

The acoustic wave probe according to the embodiment of the present invention can be produced by a conventional method, except that the acoustic matching sheet according to the embodiment of the present invention is used. That is, the manufacturing method of an acoustic wave probe according to the embodiment of the present invention includes forming an acoustic matching layer on a piezoelectric element using the acoustic matching sheet according to the embodiment of the present invention. The piezoelectric element can be provided on the backing material by a conventional method.

In addition, an acoustic lens is formed on the acoustic matching layer by a conventional method using a material for forming an acoustic lens.

### [Acoustic wave measurement apparatus]

An acoustic wave measurement apparatus according to an embodiment of the present invention includes the acoustic wave probe according to the embodiment of the present invention. The acoustic wave measurement apparatus has a function of displaying the signal intensity of a signal received by the acoustic wave probe and imaging the signal.

It is also preferable that the acoustic wave measurement apparatus according to the embodiment of the present invention is an ultrasonic measurement apparatus using an ultrasound probe.

### Examples

The present invention will be described in more detail based on Examples in which an ultrasonic wave is used as an acoustic wave. The present invention is not limited to Examples except as specified in the present invention.

In the following, the blending amount of the component means a blending amount of the component itself. That is, in a case where the raw material contains a solvent, the blending amount is an amount excluding the solvent. The acoustic wave in the present invention is not limited to the ultrasonic wave, and any acoustic wave of an audible frequency may be used as long as an appropriate frequency is selected in accordance with a test object, measurement conditions, and the like. Hereinafter, room temperature means 25°C.

[Synthesis Example]

### <1> Preparation of composition for acoustic matching layer material

### (1) Preparation of composition for acoustic matching layer material used in Example 1

A composition for an acoustic matching layer material, having composition shown in Table 1-1, was prepared.

Specifically, 86 parts by mass of metal particles (iron (Fe) (OM (trade name, manufactured by BASF, particle size: 5 µm)), 10 parts by mass of an epoxy resin (1-3) ("SUMI-EPOXY ELM-120" (trade name) (trade name) manufactured by Sumitomo Chemical Company, epoxy equivalent weight: 92)), and 2.9 parts by mass of a curing agent (2) (metaphenylene diamine, manufactured by FUJIFILM Wako Pure Chemical Corporation) were mixed by a rotation and revolution device (trade name: ARV-310, manufactured by THINKY CORPORATION) to prepare a composition for an acoustic matching layer material used in Example 1.

### (2) Preparation of compositions for acoustic matching layer material used in Examples 2 to 34 and Comparative Examples 1 to 4

Compositions for forming an acoustic matching layer material, used in Examples 2 to 34 and Comparative Examples 1 to 4, were prepared in the same manner as in the composition for forming an acoustic matching layer used in Example 1, except that composition was changed as compositions shown in Tables 1-1 to 1-4 below (hereinafter, Tables 1-1 to 1-4 are collectively referred to as Table 1).

### <2> Production of acoustic matching sheet (sheet-like acoustic matching layer material)

### (1) Production of acoustic matching sheet of Example 1

The composition for an acoustic matching layer material used in Example 1 was poured into a circular mold having a diameter of 40 mm and a depth of 3mm, and cured at 80°C for 18 hours and then at 150°C for 1 hour to produce a circular sheet-like acoustic matching layer material having a diameter of 40 mm and a thickness of 3 mm. The sheet was cut into three circular acoustic matching sheets having a diameter of 40 mm and a thickness of 1 mm with a dicer, and one acoustic matching sheet (thickness: 1 mm) in the middle was used for the following measurements.

### (2) Production of acoustic matching sheets of Examples 2 to 34 and Comparative Examples 1 to 4

An acoustic matching sheet (thickness: 1 mm; one acoustic matching sheet in the middle cut into three pieces) was produced in the same manner as the acoustic matching sheet used in Example 1, except that the compositions for an acoustic matching layer material, used in Examples 2 to 34 and Comparative Examples 1 to 4, were used instead of the composition for an acoustic matching layer material used in Example 1, and was used in the following measurements.

### [Test Example 1] Measurement of longitudinal wave acoustic velocity

The ultrasonic longitudinal wave acoustic velocity was measured at 25°C using a sing-around acoustic velocity measurement apparatus (manufactured by Ultrasonic Engineering Co., Ltd., trade name: "UVM-2 model") according to JIS Z2353 (2003). With regard to the circular acoustic matching sheet having a diameter of 40 mm and a thickness of 1 mm obtained above, for three circular regions having a diameter of 15 mm that do not overlap one another, the entire inside of these three circular regions (small probe size of a single channel) was measured. The arithmetic mean value of the longitudinal wave acoustic velocity in the above three circular regions was calculated, and evaluated based on the following evaluation standard. An evaluation of A to C is acceptable in the present test. In a case of being D, it was difficult to achieve a desired high acoustic impedance assumed by the present invention.

### -Evaluation standard-

A: 2,900 [m/sec] or more
B: 2,800 [m/sec] or more and less than 2,900 [m/sec]
C: 2,700 [m/sec] or more and less than 2,800 [m/sec]
D: less than 2,700 [m/sec]

### [Test Example 2] Measurement of density and measurement of acoustic impedance

With regard to the circular acoustic matching sheet having a diameter of 40 mm and a thickness of 1 mm obtained above, a 9 mm × 9 mm test piece was cut out from each of the three circular regions in which the longitudinal wave acoustic velocity was measured above. A density of each cut sample at 25°C was measured using an electronic hydrometer (manufactured by Alfa Mirage Co., Ltd., trade name: "SD-200L") in accordance with the density measurement method of Method A (underwater substitution method) described in JIS K7112 (1999), and the arithmetic mean value of densities in the three circular regions was obtained. An acoustic impedance was calculated from a product of the density obtained as described above and the above-described longitudinal wave acoustic velocity (arithmetic mean value of density × arithmetic mean value of longitudinal wave acoustic velocity), was evaluated based on the following evaluation standard. An evaluation of A, B, or C is acceptable in the present test.

### -Evaluation standard-

A: 13 Mrayl or more and less than 18
B: 12 Mrayl or more and less than 13 Mrayl
C: 10 Mrayl or more and less than 12 Mrayl
D: less than 10 Mrayl

### [Test Example 3] Presence or absence of air bubbles

Four side surfaces of the 9 mm × 9 mm test piece used in Test Example 2 were observed with an optical microscope at a magnification of 200 times, and the number of air bubbles was counted. A value obtained by dividing the total number of air bubbles by 4 was evaluated based on the following evaluation standard. An evaluation of A, B, or C is acceptable in the present test.

### -Evaluation standard-

A: air bubble was not found.
B: the number of air bubbles was 1 to 3.
C: the number of air bubbles was 4 to 10.
D: the number of air bubbles was 11 or more.

### [Test Example 4] Tensile test

The acoustic matching sheet (thickness: 1 mm) produced above was measured at room temperature using a TENSILON UNIVERSAL MATERIAL TESTING INSTRUMENT (trade name: RTF-1210, manufactured by A&D Company, Limited). An evaluation of A, B, or C is acceptable in the present test.

### -Evaluation standard-

A: tensile strength was 30 MPa or more.
B: tensile strength was 20 MPa or more and less than 30 MPa.
C: tensile strength was 10 MPa or more and less than 20 MPa.
D: tensile strength was less than 10 MPa.

**[Table 1-1]**

| | | | 1 | 2 | 3 | 4 | 5 | 6 | 7 | 8 | 9 | 10 |
|---|---|---|---|---|---|---|---|---|---|---|---|---|
| Epoxy resin (A) | Epoxy equivalent weight | | | | | | | | | | | |
| (1-3) | 92 | | 10 | | | | | | | | | |
| (1-5) | 97 | | | 10 | | | | | | | | |
| (1-6) | 92 | | | | 10 | 10 | 10 | 10 | 10 | 10 | 10 | 10 |
| (1-13) | 90 | | | | | | | | | | | |
| (2-1) | 136 | | | | | | | | | | | |
| (2-2) | 127 | | | | | | | | | | | |
| (2-5) | 99 | | | | | | | | | | | |
| (2-7) | 111 | | | | | | | | | | | |
| (3-2) | 106 | | | | | | | | | | | |
| (4-4) | 139 | | | | | | | | | | | |
| (4-5) | 113 | | | | | | | | | | | |
| (5-1) | 90 | | | | | | | | | | | |
| X-1 | 170 | | | | | | | | | | | |
| X-2 | 153 | | | | | | | | | | | |

| Curing agent (B-1) and curing catalyst (B-2) | Active hydrogen equivalent weight | | | | | | | | | | | |
|---|---|---|---|---|---|---|---|---|---|---|---|---|
| (1) | 43 | | | | 4.6 | | | | | | | |
| (2) | 27 | | 2.9 | 2.8 | | 2.9 | | | | | | |
| (3) | 27 | | | | | | 2.9 | | | | | |
| (4) | 50 | | | | | | | 5.4 | | | | |
| (5) | 67 | | | | | | | | 7.3 | | | |
| (7) | 40 | | | | | | | | | 4.3 | | |
| (8) | - | | | | | | | | | | 0.5 | |
| (9) | 49 | | | | | | | | | | | 5.3 |
| (12) | 72 | | | | | | | | | | | |
| (13) | - | | | | | | | | | | | |
| (14) | 154 | | | | | | | | | | | |
| X-3 | 95 | | | | | | | | | | | |
| | | | | | | | | | | | | |

| Metal particles (C) | Particle size | Density | | | | | | | | | | |
|---|---|---|---|---|---|---|---|---|---|---|---|---|
| Fe | 10 µm | 7.9 | | | | | | | | | | |
| Fe | 5 µm | 7.9 | 86 | 86 | 98 | 86 | 86 | 103 | 115 | 96 | 70 | 102 |
| Fe | 2.5 µm | 7.9 | | | | | | | | | | |
| Cu | 6 µm | 8.9 | | | | | | | | | | |
| CuO | 3 µm | 6.3 | | | | | | | | | | |
| TiC | 5 µm | 4.9 | | | | | | | | | | |
| | | | | | | | | | | | | |

| Particles (D) | Particle size | Density | | | | | | | | | | |
|---|---|---|---|---|---|---|---|---|---|---|---|---|
| SiC | 3 µm | 3.2 | | | | | | | | | | |
| Al₂O₃ | 3 µm | 4.0 | | | | | | | | | | |
| SiO₂ | 3 µm | 2.7 | | | | | | | | | | |
| Content of epoxy resin (A) (% by mass) | | | 10.1 | 102 | 8.9 | 10.1 | 10.1 | 8.4 | 7.5 | 9.1 | 12.4 | 8.5 |
| Content of curing agent (B) (% by mass) | | | 2.9 | 2.8 | 4.1 | 2.9 | 2.9 | 4.6 | 5.5 | 3.9 | 0.6 | 4.5 |
| Content of metal particles (C) (% by mass) | | | 87.0 | 87.0 | 87.0 | 87.0 | 87.0 | 87.0 | 87.0 | 87.0 | 87.0 | 87.0 |
| Content of metal particles (C) (% by volume) | | | 50.4 | 50.4 | 50.4 | 50.4 | 50.4 | 50.4 | 50.4 | 50.4 | 50.4 | 50.4 |
| Content of particles (D) (% by mass) | | | 0 | 0 | 0 | 0 | 0 | 0 | 0 | 0 | 0 | 0 |
| Test Example (1) | | | A | A | B | A | A | A | A | A | B | A |
| Test Example (2) | | | A | B | B | A | A | B | B | B | B | B |
| Test Example (3) | | | A | A | A | A | B | A | A | A | A | A |
| Test Example (4) | | | C | C | C | B | B | C | B | B | C | B |

**[Table 1-2]**

| | | | 11 | 12 | 13 | 14 | 15 | 16 | 17 | 18 | 19 | 20 |
|---|---|---|---|---|---|---|---|---|---|---|---|---|
| Epoxy resin (A) | Epoxy equivalent weight | | | | | | | | | | | |
| (1-3) | 92 | | | | | | | | | | | |
| (1-5) | 97 | | | | | | | | | | | |
| (1-6) | 92 | | 10 | 10 | 10 | | | | | | | |
| (1-13) | 90 | | | | | 10 | | | | | | |
| (2-1) | 136 | | | | | | 10 | | | | | |
| (2-2) | 127 | | | | | | | 10 | | | | |
| (2-5) | 99 | | | | | | | | 10 | | | |
| (2-7) | 111 | | | | | | | | | 10 | | |
| (3-2) | 106 | | | | | | | | | | 10 | |
| (4-4) | 139 | | | | | | | | | | | 10 |
| (4-5) | 113 | | | | | | | | | | | |
| (5-1) | 90 | | | | | | | | | | | |
| X-1 | 170 | | | | | | | | | | | |
| X-2 | 153 | | | | | | | | | | | |

| Curing agent (B-1) and curing catalyst (B-2) | Active hydrogen equivalent weight | | | | | | | | | | | |
|---|---|---|---|---|---|---|---|---|---|---|---|---|
| (1) | 43 | | | | | | | | | | | |
| (2) | 27 | | | | | 3.0 | 2.0 | 2.1 | 2.7 | 2.4 | 2.6 | 1.9 |
| (3) | 27 | | | | | | | | | | | |
| (4) | 50 | | | | | | | | | | | |
| (5) | 67 | | | | | | | | | | | |
| (7) | 40 | | | | | | | | | | | |
| (8) | -- | | | | | | | | | | | |
| (9) | 49 | | | | | | | | | | | |
| (12) | 72 | | 7.8 | | | | | | | | | |
| (13) | - | | | 0.5 | | | | | | | | |
| (14) | 154 | | | | 16.7 | | | | | | | |
| X-3 | 95 | | | | | | | | | | | |
| | | | | | | | | | | | | |

| Metal particles (C) | Particle size | Density | | | | | | | | | | |
|---|---|---|---|---|---|---|---|---|---|---|---|---|
| Fe | 10 µm | 7.9 | | | | | | | | | | |
| Fe | 5 µm | 7.9 | 119 | 70 | 179 | 87 | 80 | 81 | 85 | 83 | 84 | 80 |
| Fe | 2.5 µm | 7.9 | | | | | | | | | | |
| Cu | 6 µm | 8.9 | | | | | | | | | | |
| CuO | 3 µm | 6.3 | | | | | | | | | | |
| TiC | 5 µm | 4.9 | | | | | | | | | | |
| | | | | | | | | | | | | |

| Particles (D) | Particle size | Density | | | | | | | | | | |
|---|---|---|---|---|---|---|---|---|---|---|---|---|
| SiC | 3 µm | 3.2 | | | | | | | | | | |
| Al₂O₃ | 3 µm | 4.0 | | | | | | | | | | |
| SiO₂ | 3 µm | 2.7 | | | | | | | | | | |
| Content of epoxy resin (A) (% by mass) | | | 7.3 | 12.4 | 4.9 | 10.0 | 10.8 | 10.7 | 10.2 | 10.5 | 10.4 | 10.9 |
| Content of curing agent (B) (% by mass) | | | 5.7 | 0.6 | 8.1 | 3.0 | 2.2 | 2.3 | 2.8 | 2.5 | 2.6 | 2.1 |
| Content of metal particles (C) (% by mass) | | | 87.0 | 87.0 | 87.0 | 87.0 | 87.0 | 87.0 | 87.0 | 87.0 | 87.0 | 87.0 |
| Content of metal particles (C) (% by volume) | | | 50.4 | 50.4 | 50.4 | 50.4 | 50.4 | 50.4 | 50.4 | 50.4 | 50.4 | 50.4 |
| Content of particles (D) (% by mass) | | | 0 | 0 | 0 | 0 | 0 | 0 | 0 | 0 | 0 | 0 |
| Test Example (1) | | | B | B | C | A | B | C | C | B | B | B |
| Test Example (2) | | | B | B | C | A | B | B | A | B | B | B |
| Test Example (3) | | | A | A | A | A | A | A | A | A | A | A |
| Test Example (4) | | | C | C | C | C | B | C | C | B | B | B |

**[Table 1-3]**

| | | | 21 | 22 | 23 | 24 | 25 | 26 | 27 | 28 | 29 | 30 |
|---|---|---|---|---|---|---|---|---|---|---|---|---|
| Epoxy resin (A) | Epoxy equivalent weight | | | | | | | | | | | |
| (1-3) | 92 | | | | | | | | | | | |
| (1-5) | 97 | | | | | | | | | | | |
| (1-6) | 92 | | | | 10 | 10 | 10 | 10 | 10 | 10 | 10 | 10 |
| (1-13) | 90 | | | | | | | | | | | |
| (2-1) | 136 | | | | | | | | | | | |
| (2-2) | 127 | | | | | | | | | | | |
| (2-5) | 99 | | | | | | | | | | | |
| (2-7) | 111 | | | | | | | | | | | |
| (3-2) | 106 | | | | | | | | | | | |
| (4-4) | 139 | | | | | | | | | | | |
| (4-5) | 113 | | 10 | | | | | | | | | |
| (5-1) | 90 | | | 10 | | | | | | | | |
| X-1 | 170 | | | | | | | | | | | |
| X-2 | 153 | | | | | | | | | | | |

| Curing agent (B-1) and curing catalyst (B-2) | Active hydrogen equivalent weight | | | | | | | | | | | |
|---|---|---|---|---|---|---|---|---|---|---|---|---|
| (1) | 43 | | | | | | | | | | | |
| (2) | 27 | | 2.4 | 3.0 | 2.9 | 2.9 | 2.9 | 2.9 | 2.9 | 2.9 | 2.9 | 2.9 |
| (3) | 27 | | | | | | | | | | | |
| (4) | 50 | | | | | | | | | | | |
| (5) | 67 | | | | | | | | | | | |
| (7) | 40 | | | | | | | | | | | |
| (8) | - | | | | | | | | | | | |
| (9) | 49 | | | | | | | | | | | |
| (12) | 72 | | | | | | | | | | | |
| (13) | - | | | | | | | | | | | |
| (14) | 154 | | | | | | | | | | | |
| X-3 | 95 | | | | | | | | | | | |
| | | | | | | | | | | | | |

| Metal particles (C) | Particle size | Density | | | | | | | | | | |
|---|---|---|---|---|---|---|---|---|---|---|---|---|
| Fe | 10 µm | 7.9 | | | 86 | | | | | | | |
| Fe | 5 µm | 7.9 | 83 | 87 | | | | | | 116 | 171 | 110 |
| Fe | 2.5 µm | 7.9 | | | | 86 | | | | | | |
| Cu | 6 µm | 8.9 | | | | | 86 | | | | | |
| CuO | 3 µm | 6.3 | | | | | | 86 | | | | |
| TiC | 5 µm | 4.9 | | | | | | | 86 | | | |
| | | | | | | | | | | | | |

| Particles (D) | Particle size | Density | | | | | | | | | | |
|---|---|---|---|---|---|---|---|---|---|---|---|---|
| SiC | 3 µm | 32 | | | | | | | | | | 6 |
| Al₂O₃ | 3 µm | 4.0 | | | | | | | | | | |
| SiO₂ | 3 µm | 2.7 | | | | | | | | | | |
| Content of epoxy resin (A) (% by mass) | | | 10.5 | 10.0 | 10.1 | 10.1 | 10.1 | 10.1 | 10.1 | 7.8 | 5.4 | 7.8 |
| Content of curing agent (B) (% by mass) | | | 2.5 | 3.0 | 2.9 | 2.9 | 2.9 | 2.9 | 2.9 | 2.2 | 1.6 | 22 |
| Content of metal particles (C) (% by mass) | | | 87.0 | 87.0 | 87.0 | 87.0 | 87.0 | 87.0 | 87.0 | 90.0 | 93.0 | 85.3 |
| Content of metal particles (C) (% by volume) | | | 50.4 | 50.4 | 50.4 | 50.4 | 47.4 | 56.0 | 62.1 | 57.8 | 66.9 | 52.4 |
| Content of particles (D) (% by mass) | | | 0 | 0 | 0 | 0 | 0 | 0 | 0 | 0 | 0 | 4.7 |
| Test Example (1) | | | B | C | A | A | A | B | B | A | A | A |
| Test Example (2) | | | B | C | A | A | A | A | B | A | A | B |
| Test Example (3) | | | A | A | A | A | C | A | A | A | C | C |
| Test Example (4) | | | B | B | C | A | A | B | B | C | C | C |

**[Table 1-4]**

| | | | 31 | 32 | 33 | 34 | c1 | c2 | c3 | c4 |
|---|---|---|---|---|---|---|---|---|---|---|
| Epoxy resin (A) | Epoxy equivalent weight | | | | | | | | | |
| (1-3) | 92 | | | | | | | | | |
| (1-5) | 97 | | | | | | | | | |
| (1-6) | 92 | | 10 | 10 | 10 | 10 | | | 10 | |
| (1-13) | 90 | | | | | | | | | |
| (2-1) | 136 | | | | | | | | | |
| (2-2) | 127 | | | | | | | | | |
| (2-5) | 99 | | | | | | | | | |
| (2-7) | 111 | | | | | | | | | |
| (3-2) | 106 | | | | | | | | | 10 |
| (4-4) | 139 | | | | | | | | | |
| (4-5) | 113 | | | | | | | | | |
| (5-1) | 90 | | | | | | | | | |
| X-1 | 170 | | | | | | 10 | | | |
| X-2 | 153 | | | | | | | 10 | | |

| Curing agent (B-1) and curing catalyst (B-2) | Active hydrogen equivalent weight | | | | | | | | | |
|---|---|---|---|---|---|---|---|---|---|---|
| (1) | 43 | | | | | | | | | |
| (2) | 27 | | 2.9 | 2.9 | 2.9 | 2.9 | 1.6 | 1.8 | 2.9 | |
| (3) | 27 | | | | | | | | | |
| (4) | 50 | | | | | | | | | |
| (5) | 67 | | | | | | | | | |
| (7) | 40 | | | | | | | | | |
| (8) | - | | | | | | | | | |
| (9) | 49 | | | | | | | | | |
| (12) | 72 | | | | | | | | | |
| (13) | - | | | | | | | | | |
| (14) | 154 | | | | | | | | | |
| X-3 | 95 | | | | | | | | | 9.0 |
| | | | | | | | | | | |

| Metal particles (C) | Particle size | Density | | | | | | | | |
|---|---|---|---|---|---|---|---|---|---|---|
| Fe | 10 µm | 7.9 | | | | | | | | |
| Fe | 5 µm | 7.9 | 112 | 115 | 112 | 112 | 78 | 79 | 108 | 127 |
| Fe | 2.5 µm | 7.9 | | | | | | | | |
| Cu | 6 µm | 8.9 | | | | | | | | |
| CuO | 3 µm | 6.3 | | | | | | | | |
| TiC | 5 µm | 4.9 | | | | | | | | |
| | | | | | | | | | | |

| Particles (D) | Particle size | Density | | | | | | | | |
|---|---|---|---|---|---|---|---|---|---|---|
| SiC | 3 µm | 32 | 4 | 1 | | | | | 8 | |
| Al₂O₃ | 3 µm | 4.0 | | | 4 | | | | | |
| SiO₂ | 3 µm | 2.7 | | | | 4 | | | | |
| Content of epoxy resin (A) (% by mass) | | | 7.8 | 7.8 | 7.8 | 7.8 | 11.2 | 11.0 | 7.8 | 6.8 |
| Content of curing agent (B) (% by mass) | | | 22 | 2.2 | 22 | 2.2 | 1.8 | 2.0 | 22 | 62 |
| Content of metal particles (C) (% by mass) | | | 86.9 | 89.2 | 86.9 | 86.9 | 87.0 | 87.0 | 83.8 | 87.0 |
| Content of metal particles (C) (% by volume) | | | 542 | 56.8 | 54.7 | 53.7 | 49.1 | 49.2 | 50.8 | 50.4 |
| Content of particles (D) (% by mass) | | | 3.1 | 0.8 | 3.1 | 3.1 | 0 | 0 | 6.2 | 0 |
| Test Example (1) | | | A | A | A | A | C | B | A | D |
| Test Example (2) | | | B | A | B | B | D | D | A | D |
| Test Example (3) | | | C | C | C | C | C | C | D | C |
| Test Example (4) | | | C | C | C | C | B | B | D | C |

### <Notes of table>

1 to 34 of top row: Examples 1 to 34
c1 to c4 of top row: Comparative Examples c1 to c4

### [Epoxy resin (A)]

### -Epoxy resin (A) used in Examples-

(1-3), (1-5), (1-6), (1-13), (2-1), (2-2), (2-5), (2-7), (3-2), (4-4), and (4-5): exemplary compounds (1-3), (1-5), (1-6), (1-13), (2-1), (2-2), (2-5), (2-7), (3-2), (4-4), and (4-5) described above

### (5-1): compound shown below

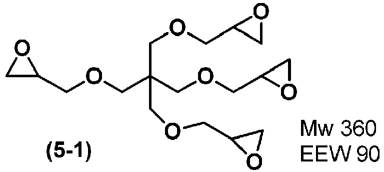

### -Epoxy resin used in Comparative Examples-

### X-1: compound shown below

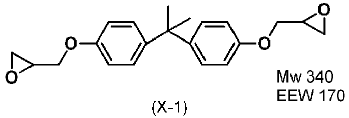

### X-2: compound shown below

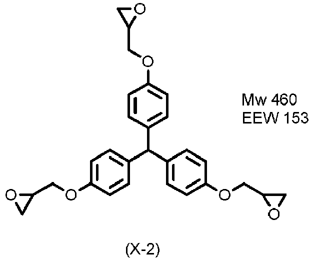

X-1 and X-2 are described in the column of the epoxy resin (A) in order to facilitate comparison between Examples and Comparative Examples.

### [Curing agent (B-1) and curing catalyst (B-2)]

(1), (2), (3), (4), (5), (7), (8), (9), (12), (13), and (14): exemplary compounds (1), (2), (3), (4), (5), (7), (8), (9), (12), (13), and (14) described above
X-3: polyamide amine LUCKAMIDE EA-330 (trade name), manufactured by DIC Corporation
X-3 is described in the column of "Curing agent (B-1) and curing catalyst (B-2)" in order to facilitate comparison between Examples and Comparative Examples.

In addition, "Content of curing agent (B) (% by mass)" means the content of the curing agent (B-1) and the curing catalyst (B-2).

### [Metal particles (C)]

Fe (particle size: 10 µm): iron powder (iron particles) (SP-I (trade name) manufactured by BASF)
Fe (particle size: 5 µm): iron powder (iron particles) (OM (trade name) manufactured by BASF)
Fe (particle size: 2.5 µm): iron powder (iron particles) (FM (trade name) manufactured by BASF)
Cu (particle size: 6 µm): copper powder (copper particles) (MA-C05K-2 (trade name) manufactured by MITSUI MINING & SMELTING CO., LTD.)
CuO (particle size: 3 µm): copper oxide powder (copper oxide particles) (FCO-500 (trade name) manufactured by FURUKAWA CHEMICALS CO., LTD.)
TiC (particle size: 5 µm): titanium carbide powder (titanium carbide particles) (manufactured by Kojundo Chemical Lab. Co., Ltd.)

### [Particles (D)]

SiC (particle size: 3 µm): silicon carbide particles (manufactured by Kojundo Chemical Lab. Co., Ltd.)
Al₂O₃ (particle size: 3 µm): alumina particles (N-9000 (trade name) manufactured by Nishimura Advanced Ceramics)
SiO₂ (particle size: 3 µm): silica particles (manufactured by COREFRONT Corporation)

From Table 1, the following was found.

With regard to the acoustic matching sheets of Comparative Examples 1 and 2, which were produced using an epoxy resin having an epoxy equivalent weight of more than 140, the curing agent (B-1), and the metal particles (C), Test Example 2 (acoustic impedance) was unacceptable.

In the acoustic matching sheet of Comparative Example 3, although the epoxy resin (A), the curing agent (B-1), and the metal particles (C) were used, since the content of the particles (D) was more than 5% by mass, a large number of air bubbles were generated and sufficient mechanical strength could not be obtained.

With regard to the acoustic matching sheet of Comparative Example 4, which was produced using the epoxy resin (A), an amine curing agent which was a polymer, and the metal particles (C), Test Example 1 (longitudinal wave acoustic velocity) and Test Example 2 (acoustic impedance) were unacceptable.

On the other hand, in the acoustic matching sheets of Examples 1 to 34 according to the embodiment of the present invention, it was found that the content of air bubbles was low, sufficient mechanical strength was obtained, high longitudinal wave acoustic velocity was exhibited, and high acoustic impedance was exhibited with a thin film shape.

The present invention has been described with the embodiments thereof, any details of the description of the present invention are not limited unless described otherwise, and it is obvious that the present invention is widely construed without departing from the gist and scope of the present invention described in the accompanying claims.

The present application claims the priority of JP2021-161985 filed in Japan on September 30, 2021, the contents of which are incorporated herein by reference, as a part of the description of the present specification.

### Explanation of References

1: acoustic lens
2: acoustic matching layer
3: piezoelectric element layer
4: backing material
7: housing
9: cord
10: ultrasound probe

## Claims

1. An acoustic matching layer material comprising:
a curing reaction product obtained by reacting an epoxy resin (A) having an epoxy equivalent weight of 140 or less with at least one of a curing agent (B-1) which is a monomer or a curing catalyst (B-2); and
metal particles (C) having a density of 4.5 g/cm³ or more and less than 10 g/cm³ at 20°C,
wherein a content of particles (D) having a density of less than 4.5 g/cm³ at 20°C is less than 5% by mass.

2. The acoustic matching layer material according to claim 1,
wherein the epoxy resin (A) is a compound represented by any one of General Formula (1), ..., or (4), 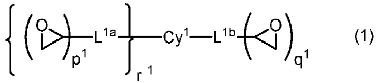
in General Formula (1), Cy¹ represents a ring, L^{1a} represents a linking group and L^{1b} represents a linking group containing a nitrogen atom, p¹ is 1 or 2, q¹ is 1 or 2, and r¹ is an integer of 1 to 3, 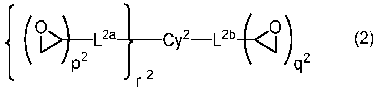
in General Formula (2), Cy² represents a ring, L^{2a} and L^{2b} represent an alkylene group, an alkanetriyl group, an oxygen atom, or a linking group obtained by combining these groups, p² is 1 or 2, q² is 1 or 2, and r² is an integer of 1 to 3, 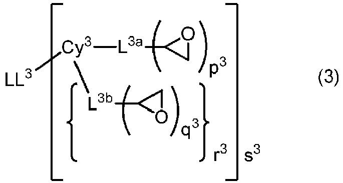
in General Formula (3), Cy³ represents a ring, L^{3a} represents a linking group containing a nitrogen atom and L^{3b} represents a linking group, LL³ represents a linking group, p³ is 1 or 2, q³ is 1 or 2, r³ is an integer of 0 to 3, and s³ is 2 or 3,
where the compound represented by General Formula (3) has three or more epoxy groups, 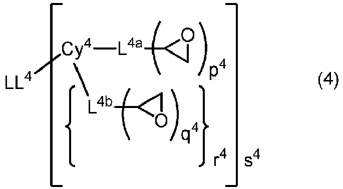
in General Formula (4), Cy⁴ represents a ring, L^{4a} and L^{4b} represent an alkylene group, an alkanetriyl group, an oxygen atom, or a linking group obtained by combining these groups, LL⁴ represents a linking group, p⁴ is 1 or 2, q⁴ is 1 or 2, r⁴ is an integer of 0 to 3, and s⁴ is 2 or 3,
where the compound represented by General Formula (4) has three or more epoxy groups.

3. The acoustic matching layer material according to claim 1 or 2,
wherein the curing agent (B-1) includes an amine compound.

4. The acoustic matching layer material according to any one of claims 1 to 3,
wherein the curing agent (B-1) includes a primary amine compound.

5. The acoustic matching layer material according to any one of claims 1 to 4,
wherein the curing agent (B-1) includes an aromatic ring-containing primary amine compound.

6. The acoustic matching layer material according to any one of claims 1 to 5,
wherein the epoxy resin (A) has an aromatic hydrocarbon ring.

7. The acoustic matching layer material according to any one of claims 1 to 6,
wherein a longitudinal wave acoustic velocity of an ultrasonic wave at 25°C is 2,700 m/sec or more.

8. The acoustic matching layer material according to any one of claims 1 to 7,
wherein an acoustic impedance at 25°C is 12 Mrayl or more.

9. An acoustic matching sheet consisting of the acoustic matching layer material according to any one of claims 1 to 8.

10. A composition for an acoustic matching layer material, which is used for obtaining the acoustic matching layer material according to any one of claims 1 to 8, the composition comprising:
an epoxy resin (A) having an epoxy equivalent weight of 140 or less;
at least one of a curing agent (B-1) which is a monomer or a curing catalyst (B-2); and
metal particles (C) having a density of 4.5 g/cm³ or more and less than 10 g/cm³ at 20°C,
wherein a content of particles (D) having a density of less than 4.5 g/cm³ at 20°C in a solid content is less than 5% by mass.

11. An acoustic wave probe comprising:
the acoustic matching sheet according to claim 9 as an acoustic matching layer.

12. An acoustic wave measurement apparatus comprising:
the acoustic wave probe according to claim 11.

13. The acoustic wave measurement apparatus according to claim 12,
wherein the acoustic wave measurement apparatus is an ultrasound diagnostic apparatus.

14. A manufacturing method of an acoustic wave probe, comprising:
forming an acoustic matching layer on a piezoelectric element using the acoustic matching layer material according to any one of claims 1 to 8.
